# FASCICULE DE BREVET EUROPEEN

(11) **EP 0 615 755 B1**
(45) Date de publication et mention de la délivrance du brevet: **15.01.2003**
(21) Numéro de dépôt: 94400597.4
(22) Date de dépôt: 18.03.1994
(51) Int. Cl.: A61K 31/445, A61P 9/10

(54) **Utilisation de dérivés de la tétrahydropyridine pour la préparation de médicaments cardioprotecteurs**
Verwendung von Tetrahydropyridinderivaten zur Herstellung von Kardioprotektiven Medikamenten
Use of tetrahydropyridine derivatives for the manufacture of cardioprotective medicaments

(30) Priorité: 18.03.1993 FR 9303149
(43) Date de publication de la demande: 21.09.1994
(73) Titulaire: SANOFI-SYNTHELABO, 75013 Paris (FR)
(72) Inventeur: Manning, Allan Stewart, B-3090 Overijse (BE); Chatelain, Pierre Paul, B-1150 Bruxelles (BE)
(74) Mandataire: Gillard, Marie-Louise

(56) Documents cités:
- EP-A- 0 498 718
- WO-A-91/03548
- MED CLIN NORTH AM (UNITED STATES), JAN 1988, VOL. 72, NO. 1, PAGE(S) 243-58, Greenberg SM et al 'Coenzyme Q10: a new drug for myocardial ischemia?'
- EUR J PHARMACOL (NETHERLANDS), JAN 7 1992, VOL. 210, NO. 1, PAGE(S) 85-90, Petty MA et al 'reperfusion injury in rats.'
- AM HEART J (UNITED STATES), JAN 1986, VOL. 111, NO. 1, PAGE(S) 95-102, Milei J et al 'Amelioration of adriamycin-induced cardiotoxicity in rabbits by prenylamine and vitamins A and E.'

## Description

La présente invention concerne l'utilisation d'une 1-[2-naphtyléthyl]-4-(3-trifluorométhylphényl)-1,2,3,6-tétrahydropyridine de formule (I) 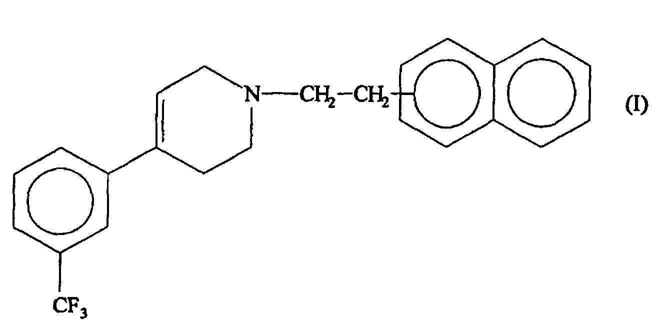 ou de ses sels d'addition avec des acides pharmaceutiquement acceptables, pour la préparation de compositions pharmaceutiques destinées au traitement de l'insuffisance cardiaque, des vasospasmes coronaires, de l'angine de poitrine, du prolapsus des valves cardiaques, à la prévention d'une deuxième attaque cardiaque, au traitement et/ou à la prophylaxie des pathologies inflammatoires cardiaques, à la prévention ou au traitement des effets secondaires de médicaments cardiotoxiques, ou au traitement de patients soumis à des opérations en cardiochirurgie.

Dans la formule (I) ci-dessus, le groupe 1-[4-(3-trifluorométhylphényl)-1,2,3,6-tétrahydropyridinyl]éthyle peut être lié en position 1- ou 2- du naphtalène.

Selon un aspect préféré de la présente invention ce groupe est lié en position 2-du naphtalène.

Les composés de formule (I), sous forme de bases libres ou de sels d'addition, ainsi que leur préparation et leur activité anorexigène ont été décrits dans la demande de brevet européen EP-A-101381. Parmi les sels d'addition pharmaceutiquement acceptables du composé de formule (I), on peut citer ceux formés avec les acides minéraux, tels que par exemple les acides chlorhydrique, bromhydrique, phosphorique ou sulfurique, ou avec les acides organiques, tels que l'acide acétique, formique, propionique, benzoïque, maléique, succinique, tartarique, fumarique, citrique, glyoxylique, aspartique, méthanesulfonique, éthanesulfonique, benzènesulfonique, p-toluènesulfonique, etc.

D'autres activités thérapeutiques des composés de formule (I) ont été décrites plus récemment, à savoir l'activité anxiolytique et antidépressive (EP-A-369887), anticonstipante (EP-A-412901), neurotrophique / neuroprotectrice (EP-A-458696) et antiradicalaire (EP-A-498718).

On a maintenant trouvé, de façon tout à fait inattendue, que les composés de formule (I) sont aussi indiqués, à de faibles doses, dans le traitement des pathologies mentionnées ci-dessus.

L'activité cardioprotectrice des composés de formule (I) a été mise en évidence à l'aide d'un test de nécrose myocardique chez le rat.

Le modèle qui a été utilisé est une modification de celui proposé par G. Rona et al., Arch. Path., 1959, 67:443-455. Il est basé sur le fait que l'administration de doses élevées d'isoprénaline provoque chez les rats des nécroses myocardiques très importantes qui ressemblent à celles qu'on retrouve chez les animaux ayant subi un infarctus myocardique spontané. Les lésions myocardiques provoquées par l'isoprénaline représentent donc un modèle très satisfaisant pour étudier les nécroses cardiaques ainsi que les facteurs qui les améliorent ou les aggravent.

### ACTIVITE CARDIOPROTECTRICE

### - Essai 1

Dans le modèle utilisé pour l'évaluation de l'activité cardioprotectrice des composés (I), le produit à tester a été administré par voie orale à différentes doses une fois par jour pendant 14 jours, la première prise deux heures avant une injection sous-cutanée unique d'isoprénaline (40 mg/kg). Le groupe témoin d'animaux reçoit seulement l'injection sous-cutanée unique d'isoprénaline (40 mg/kg). A la fin de cette période, les coeurs ont été prélevés, pesés et fixés pour l'étude histologique. L'évaluation microscopique des lésions myocardiques et du degré de réparation tissulaire a été faite en aveugle par la méthode de la grille. La présence de chaque type cellulaire (myocytes sains ou nécrotiques, cellules inflammatoires, fibroblastes) ainsi que la présence de fibres collagènes est exprimée en pourcent. La comparaison entre groupe témoin et groupes traités a été effectuée en calculant le "t" de Student, pour des valeurs indépendantes, sur les répartitions respectives de ces différents types cellulaires.
On a pu ainsi observer que, chez les animaux traités avec des doses, variant entre 1 et 10 mg/kg du chlorhydrate de 1-[2-(2-naphtyl)éthyl]-4-(3-trifluorométhylphényl)-1,2,3,6-tétrahydropyridine, la proportion d'atteinte myocardique induite par l'isoprénaline (tissu nécrotique, présence de cellules inflammatoires, pycnose) est significativement inférieure à celle des témoins et que, de même, la proportion des processus correspondant à la réparation (présence de fibroblastes/fibrocytes et de fibres collagènes) est significativement inférieure à celle des témoins.

### - Essai 2

Afin de vérifier si l'action cardioprotectrice se manifeste précocement sur les conséquences immédiates de l'isoprénaline, on a effectué un test selon la méthode décrite dans l'essai 1 en effectuant l'étude histologique des coeurs le 4ème jour après l'injection d'isoprénaline et donc après seulement 3 jours de traitement.

On a pu constater que les composés de formule (I) exercent aussi un important effet protecteur dans ce test de traitement réduit, ce qui démontre que l'activité cardioprotectrice est déjà significative le troisième jour du traitement.

Cet effet cardioprotecteur n'est pas lié à un effet direct sur la fonction cardiaque : la perfusion du coeur isolé de rat avec 1µM du même composé ne change ni la fréquence cardiaque, ni la pression ventriculaire, ni la dP/dt max, ni le débit coronaire.

Ainsi, la présente invention se rapporte à l'utilisation des composés de formule (I) et de leurs sels pharmaceutiquement acceptables pour la préparation de compositions pharmaceutiques destinées au traitement de l'insuffisance cardiaque, des vasospasmes coronaires, de l'angine de poitrine, du prolapsus des valves cardiaques. De plus, grâce à leur intervention bénéfique dans les processus inflammatoires, les composés de formule (I) peuvent être indiqués dans les pathologies inflammatoires cardiaques, par exemple dans le traitement et/ou la prophylaxie de la péricardite et l'endocardite.

Les composés de formule (I) peuvent également être utilisés pour la préparation de médicaments destinés à protéger le coeur qui a déjà subi une attaque, plus particulièrement pour la préparation de médicaments destinés à prévenir une deuxième attaque cardiaque.

En outre, les composés de formule (I) et leurs sels pharmaceutiquement acceptables peuvent être utilisés pour la préparation de compositions pharmaceutiques destinées à prévenir ou à traiter les effets secondaires de cardiotoxicité de médicaments tels que les anticancéreux, par exemple ceux de la famille des anthracyclines, comme la doxorubicine, ceux de la famille des dérivés du platine, comme le cisplatine, ou ceux d'intercalants comme le chlorure d'elliptinium, les catécholamines naturelles ou synthétiques, l'excès de caféine.

Enfin, les composés de formule (I) et leurs sels pharmaceutiquement acceptables peuvent être utilisés pour la préparation de compositions pharmaceutiques destinées au traitement des personnes obèses pour diminuer les risques d'attaques cardiaques ou bien au traitement de patients soumis à des opérations en cardiochirurgie, par exemple, à une angioplastie transluminale ou à une greffe coronaire.

Les composés de formule (I) ou leurs sels d'addition pharmaceutiquement acceptables sont formulés dans des compositions pour l'administration par voie orale, parentérale, sublinguale ou transdermique. La quantité de principe actif à administrer dépend de la nature et de la gravité des affections à traiter ainsi que du poids du malade, et, dans le cas d'administration conjointe avec des médicaments cardiotoxiques, du dosage de ces derniers.

Les compositions pharmaceutiques de l'invention renferment une quantité efficace d'au moins un produit choisi parmi les composés de formule (I) et leurs sels d'addition pharmaceutiquement acceptables, en association avec un véhicule pharmaceutique inerte. Les doses unitaires comprennent de 2 à 500 mg, avantageusement de 5 à 250 mg, de préférence 5 à 150 mg, par exemple, 5, 10, 30, 50, 70, 90, 100, 110, 130 et 150 mg, de principe actif. Ces doses unitaires seront administrées normalement une ou plusieurs fois par jour, par exemple 2 ou 3 fois par jour, de préférence une ou deux fois par jour, la dose globale chez l'homme variant entre 2 et 500 mg par jour, par exemple de 5 à 250 mg, de préférence de 10 à 150 mg par jour.

Pour l'administration par voie orale ou sublinguale, le principe actif est formulé en particulier dans des comprimés, simples ou dragéifiés, des gélules granulées éventuellement à libération retardée, des gouttes ou encore des liposomes. Pour l'administration par voie intraveineuse, sous-cutanée ou intramusculaire, on prépare des lyophilisats, des solutions stériles ou stérilisables, tandis que l'on peut préparer des patches classiques pour l'administration par voie transdermique.

Les compositions pharmaceutiques selon la présente invention peuvent être préparées selon des méthodes usuelles, telles que celles décrites dans EP-101381 ou dans le Remington's Pharmaceutical Sciences, 18th Ed., Mack Publ. Co. Le principe actif peut être incorporé dans des excipients habituellement employés dans ces compositions pharmaceutiques, tels que le talc, la gomme arabique, le lactose, la cellulose, la silice, le mannitol, l'amidon, le stéarate de magnésium, les véhicules aqueux ou non, les corps gras d'origine animale ou végétale, les dérivés paraffiniques, les glycols, les différents agents mouillants, dispersants, ou émulsifiants, les conservateurs, etc.

Dans les compositions pharmaceutiques selon la présente invention le principe actif de formule (I) peut aussi être sous forme de complexe d'inclusion dans des cyclodextrines, leurs éthers ou leurs esters.

Quand les composés de formule (I) ou leurs sels pharmaceutiquement acceptables sont utilisés pour le traitement des conditions pathologiques cardiaques, les compositions pharmaceutiques les contenant peuvent aussi avantageusement contenir un ou plusieurs autres médicaments connus et communément utilisés dans le traitement de ces conditions pathologiques. Parmi ces autres médicaments on peut citer les inhibiteurs de l'enzyme de conversion de l'angiotensine, tels que par exemple le captopril, l'énalapril, le fosinopril, le quinapril et le ramipril ; les antagonistes calciques, tels que par exemple le diltiazem, le verapamil, la nifédipine, la nicardipine et l'amlodipine ; les vasodilateurs nitrés, tels que par exemple le nicorandil, la trinitrine et l'isosorbide mono- ou di-nitrée ; les β-bloquants, tels que le propranolol, le sotalol, le métoprolol et le nadolol ; les glucosides cardiaques, tels que la digitoxine, la digoxine et la métildigoxine ; les antagonistes des récepteurs au thromboxane A₂.

Quand les composés de formule (I) sont utilisés dans le contexte d'une condition pathologique présentant des risques pour le coeur comme résultat de cette condition, ils seront administrés pendant une période qui sera dictée par l'existence de cette condition ou du danger que cette condition puisse se répéter avec des effets néfastes.

Quand les composés de formule (I) sont utilisés dans le traitement avec des médicaments ayant des effets secondaires de cardiotoxicité, la période d'administration des composés (I) dépendra de la période d'administration desdits médicaments.

Quand les composés de formule (I) ou leurs sels pharmaceutiquement acceptables sont utilisés dans le cas du traitement avec un médicament cardiotoxique, ils peuvent être formulés séparément du médicament cardiotoxique et ils pourront être administrés avant ou en même temps avec le médicament cardiotoxique ou, si nécessaire, même après le médicament cardiotoxique.

De toutes façons, si on le désire, les composés de formule (I) et le médicament cardiotoxique peuvent être formulés dans la même composition pharmaceutique.

Les exemples suivants illustrent mieux l'invention sans toutefois la limiter ; dans ces exemples on décrit des compositions pharmaceutiques contenant, en tant que principe actif, la 1-[2-(2-naphtyl)éthyl]-4-(3-trifluorométhylphényl)-1,2,3,6-tétrahydropyridine (indiqué pour commodité de lecture, comme Composé A), sous forme de son chlorhydrate.

### EXEMPLE 1

On prépare des gélules de gélatine et dioxyde de titane, ayant la composition unitaire suivante :
- Chlorhydrate du Composé A 54,8 mg
- Amidon de maïs modifié 104,72 mg
- Cellulose microcristalline 15,00 mg
- Silice colloïdale anhydre 0,16 mg
- Stéarate de magnésium 0,32 mg

### EXEMPLE 2

On prépare des gélules de gélatine et dioxyde de titane, ayant la composition unitaire suivante :
- Chlorhydrate du Composé A 5,48 mg
- Amidon de maïs modifié 142,92 mg
- Cellulose microstalline 26,00 mg
- Silice colloïdale anhydre 0,20 mg
- Stéarate de magnésium 0,40 mg

### EXEMPLE 3

On prépare un lyophilisat de composition suivante :
- Chlorhydrate du Composé A 13,2 mg
- Acide citrique 240,00 mg
- Tween® 80 301,50 mg
- Mannitol 1,2001 g
- NaOH 1 N 685,00 mg
- Eau PPI q.s.p. 30,00 g

### Méthode de préparation :

On dissout l'acide citrique dans l'eau PPI (pour préparations injectables) et on y ajoute le chlorhydrate du Composé A. Après 24 heures, les autres composants sont ajoutés et on procède à la lyophilisation selon les méthodes usuelles.

### EXEMPLE 4

En suivant la méthode de l'exemple 3, on prépare un lyophilisat ayant la composition suivante :
- Chlorhydrate du Composé A 33,20 mg
- Hydroxypropylbétacyclodextrine 751,20 mg
- Acide citrique 2,10 mg
- Mannitol 1509,10 mg
- Eau PPI q.s.p. 30,00 g

## Revendications

1. Utilisation d'un composé de formule (I) 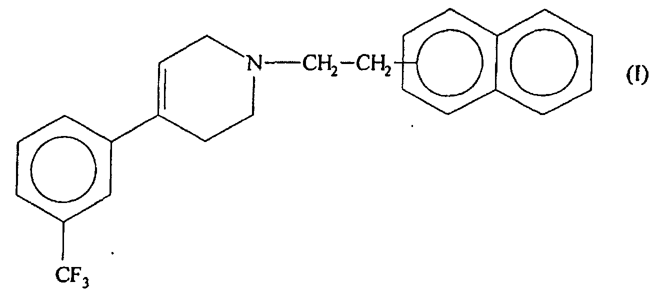 ou d'un de ses sels pharmaceutiquement acceptables, pour la préparation de compositions pharmaceutiques destinées au traitement de l'insuffisance cardiaque, des vasospasmes coronaires, de l'angine de poitrine, du prolapsus des valves cardiaques, à la prévention d'une deuxième attaque cardiaque, au traitement et/ou à la prophylaxie des pathologies inflammatoires cardiaques, à la prévention ou au traitement des effets secondaires de médicaments cardiotoxiques, ou au traitement de patients soumis à des opérations en cardiochirurgie..

2. Utilisation selon la revendication 1, **caractérisée en ce que** le composé de formule (I) est la 1-[2-(2-naphtyl)éthyl]-4-(3-trifluorométhylphényl)-1,2,3,6-tétrahydropyridine ou un de ses sels pharmaceutiquement acceptables.

3. Utilisation selon la revendication 2, **caractérisée en ce que** le composé de formule (I) est le chlorhydrate de 1-[2-(2-naphtyl)éthyl]-4-(3-trifluorométhylphényl)-1,2,3,6-tétrahydropyridine.

4. Utilisation selon l'une des revendications 1 à 3, **caractérisée en ce que** la composition pharmaceutique est destinée à la prévention ou au traitement des effets secondaires d'un médicament anticancéreux.

5. Utilisation selon la revendication 4, **caractérisée en ce que** l'anticancéreux appartient à la famille des anthracyclines.

## Claims

1. Use of a compound of formula (I) 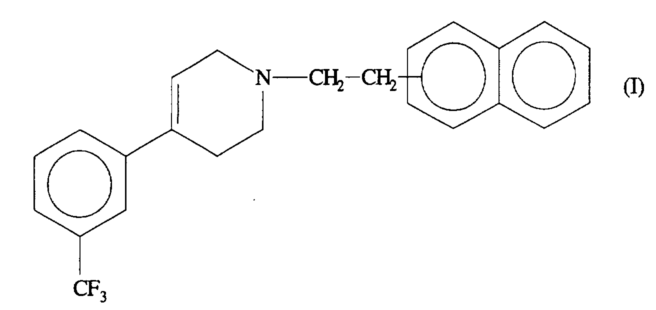 or of a pharmaceutically acceptable salt thereof, for the preparation of pharmaceutical compositions intended for the treatment of heart failure, coronary vasospasm, angina pectoris, valvular heart prolapse, for the prevention of a second heart attack, for the treatment and/or prophylaxis of heart inflammatory pathologies, for the prevention or treatment of side-effects caused by cardiotoxic medicaments, or for the treatment of patients subjected to heart surgery.

2. Use according to claim 1 **characterized in that** the compound of formula (I) is 1-[2-(2-naphtyl)ethyl]-4-(3-trifluoromethylphenyl)-1,2,3,6-tetrahydropyridine or a pharmaceutically acceptable salt thereof.

3. Use according to claim 2 **characterized in that** the compound of formula (I) is 1-[2-(2-naphtyl)ethyl]-4-(3-trifluoromethylphenyl)-1,2,3,6-tetrahydropyridine hydrochloride.

4. Use according to one of claims 1 to 3, **characterized in that** the pharmaceutical composition is intended for the prevention or treatment of side effects caused by an anticancer medicament.

5. Use according to claim 4, **characterized in that** the anticancer medicament belongs to the anthracycline family.

## Patentansprüche

1. Verwendung einer Verbindung der Formel (I) 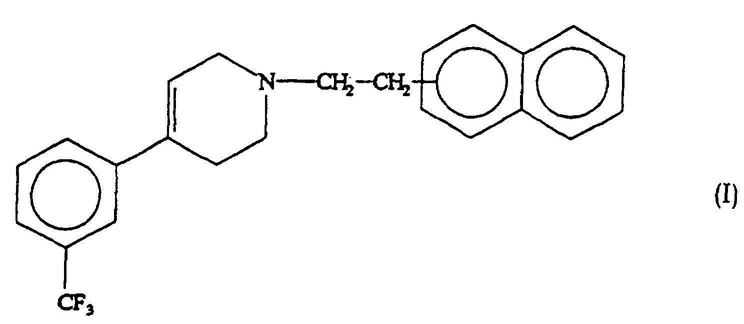 oder eines ihrer pharmazeutisch akzeptablen Salze für die Herstellung pharmazeutischer Zusammensetzungen, die für die Behandlung der Herzinsuffizienz, von koronaren Vasospasmen, der Angina pectoris, des Herzklappenprolapses, zur Verhinderung einer zweiten Herzattacke, für die Behandlung von und/oder zur Vorbeugung vor Herzentzündungserkrankungen, zur Verhinderung oder Behandlung der Nebenwirkungen kardiotoxischer Arzneimittel oder für die Behandlung von Patienten, die kardiochirurgischen Operationen unterzogen werden, vorgesehen sind.

2. Verwendung nach Anspruch 1, **dadurch gekennzeichnet, daß** die Verbindung der Formel (I) das 1-[2-(2-Naphthyl)-ethyl]-4-(3-trifluormethylphenyl)-1,2,3,6-tetrahydropyridin oder eines seiner pharmazeutisch akzeptablen Salze ist.

3. Verwendung nach Anspruch 2, **dadurch gekennzeichnet, daß** die Verbindung der Formel (I) das Hydrochlorid von 1-[2-(2-Naphthyl)-ethyl]-4-(3-trifluormethylphenyl)-1,2,3,6-tetrahydropyridin ist.

4. Verwendung nach einem der Ansprüche 1 bis 3, **dadurch gekennzeichnet, daß** die pharmazeutische Zusammensetzung für die Verhinderung oder die Behandlung der Nebenwirkungen eines Antikrebsmittels vorgesehen ist.

5. Verwendung nach Anspruch 4, **dadurch gekennzeichnet, daß** das Antikrebsmittel zur Familie der Anthracycline gehört.
